(19) Europäisches Patentamt
European Patent Office
Office européen des brevets

(11) EP 0 855 896 B1

(12) **FASCICULE DE BREVET EUROPEEN**

(45) Date de publication et mention de la délivrance du brevet:
**05.09.2001 Bulletin 2001/36**

(21) Numéro de dépôt: **96933485.3**

(22) Date de dépôt: **03.10.1996**

(51) Int Cl.[7]: **A61K 7/06**, A61K 7/13

(86) Numéro de dépôt international:
**PCT/FR96/01547**

(87) Numéro de publication internationale:
**WO 97/15271 (01.05.1997 Gazette 1997/19)**

(54) **PROCEDE DE TEINTURE D'OXYDATION DES FIBRES KERATINIQUES ET COMPOSITION MISE EN OEUVRE AU COURS DE CE PROCEDE**

VERFAHREN ZUR OXYDATIVEN FÄRBUNG DER HAARE UND BEI DER DURCHFÜHRUNG DES VERFAHRENS VERWENDETE ZUSAMMENSETZUNG

METHOD FOR OXIDATION DYEING KERATIN FIBRES AND COMPOSITION THEREFOR

(84) Etats contractants désignés:
**AT BE CH DE DK ES FR GB GR IE IT LI LU MC NL PT SE**

(30) Priorité: **20.10.1995 FR 9512385**

(43) Date de publication de la demande:
**05.08.1998 Bulletin 1998/32**

(73) Titulaire: **L'OREAL**
**75008 Paris (FR)**

(72) Inventeurs:
- **LAURENT, Florence**
  **F-92600 Asnières (FR)**
- **BRAIDA-VALERIO, Damarys**
  **F-75004 Paris (FR)**

(74) Mandataire: **Tezier Herman, Béatrice**
**L'OREAL-DPI**
**6 rue Bertrand Sincholle**
**92585 Clichy Cédex (FR)**

(56) Documents cités:
**EP-A- 0 647 617**
**FR-A- 2 679 770**
**FR-A- 2 718 960**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 855 896 B1

Printed by Jouve, 75001 PARIS (FR)

## Description

La présente invention a pour objet un procédé de teinture des fibres kératiniques et en particulier des fibres kératiniques humaines telles que les cheveux, mettant en oeuvre une composition tinctoriale contenant au moins un colorant d'oxydation et une composition oxydante contenant au moins un agent oxydant, ladite composition tinctoriale et/ou ladite composition oxydante comprenant au moins au moins un composé de type céramide.

L'invention a également pour objet la composition tinctoriale contenant au moins un colorant d'oxydation et au moins un composé de type céramide mise en oeuvre au cours de ce procédé.

Il existe principalement deux types de coloration des fibres kératiniques. La coloration directe mettant en oeuvre des colorants directs et/ou des pigments qui sont des molécules colorées conférant aux fibres une couleur temporaire s'estompant après quelques shampooings et la coloration dite "coloration d'oxydation" mettant en oeuvre des précurseurs de colorants d'oxydation et un agent oxydant qui confère aux fibres une couleur tenace.

Dans le cadre de la coloration d'oxydation, on met généralement en oeuvre des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliniques.

La variété des molécules mises en jeu au niveau des bases d'oxydation et des coupleurs permet l'obtention d'une riche palette de couleurs.

La coloration d'oxydation permet, comme on l'a vu précédemment, de teindre les cheveux de manière durable, cependant elle est généralement réalisée dans des conditions entraînant une dégradation non négligeable des fibres kératiniques. En effet, la présence d'un agent oxydant et d'un milieu généralement très alcalin entraîne une dégradation des fibres kératiniques, rendant celles-ci souvent rêches et cassantes.

La coloration dite "permanente" obtenue grâce aux colorants d'oxydation doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Or, la demanderesse vient maintenant de découvrir, de façon totalement inattendue et surprenante, que l'utilisation de composés de type céramide dans des compositions pour la teinture d'oxydation des fibres kératiniques permet de conférer à ces fibres une coloration résistant mieux au cours du temps aux diverses agressions extérieures qu'elles peuvent subir.

Cette découverte est à la base de la présente invention.

La présente invention a donc pour objet un procédé de teinture des fibres kératiniques et en particulier des fibres kératiniques humaines telles que les cheveux, caractérisé par le fait qu'on applique sur ces fibres :

- au moins une composition tinctoriale contenant, dans un milieu approprié pour la teinture, au moins une base d'oxydation choisie parmi les bis-phénylalkylènediamines, les para-aminophénols, les ortho-aminophénols, les bases hétérocycliques et les paraphénylènediamines de formule (I) suivante, et les sels d'addition avec un acide de ces composés :

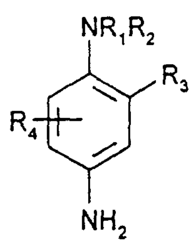

(I)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, phényle ou 4'-aminophényle,

$R_2$ représente un atome d'hydrogène, un radical alkyle en $C_1$-$C_4$, monohydroxyalkyle en $C_1$-$C_4$ ou polyhydroxyalkyle en $C_2$-$C_4$,
$R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$ ou hydroxyalcoxy en $C_1$-$C_4$,
$R_4$ représente un atome d'hydrogène ou un radical alkyle en $C_1$-$C_4$ ;

- la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée ; ladite composition tinctoriale et/ou ladite composition oxydante contenant au moins un composé de type céramide.

Les colorations obtenues selon le procédé de teinture conforme à l'invention présentent d'excellentes propriétés de résistances à la fois vis à vis des agents atmosphériques tels que la lumière et les intempéries et vis à vis de la transpiration et des différents traitements que peuvent subir les cheveux (lavages, déformations permanentes). De plus, les fibres ainsi colorées sont moins altérées par le processus de coloration d'oxydation et restent plus douces et moins cassantes que lorsqu'un procédé de teinture ne mettant pas en oeuvre de composé de type céramide est utilisé.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Les composés de type céramide pouvant être utilisés dans la composition tinctoriale et/ou dans la composition oxydante sont connus en eux-mêmes. Ils incluent les céramides proprement dits, les glycocéramides, les pseudocéramides et les néocéramides, naturelles ou synthétiques.

Des composés de type céramide sont par exemple décrits dans les demandes de brevet DE-A-4 424 530, DE-A-4 424 533, DE-A-4 402 929, DE-A-4 420 736, WO 95 / 23 807, WO 94 / 07 844, EP-A-0 646 572, WO 95/16 665, FR-A-2 673 179, EP-A-0 227 994, WO 94 / 07 844, WO 94 / 24 097 et WO 94 /10 131 dont les enseignements sont ici inclus à titre de référence.

Les composés de type céramide, naturels ou synthétiques, utilisables selon la présente invention répondent préférentiellement à la formule générale (II) :

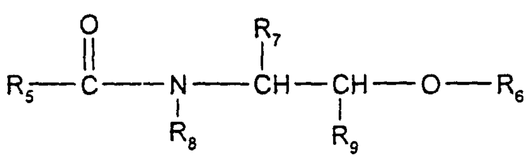 (II)

dans laquelle :

- $R_5$ désigne :
  - soit un radical hydrocarboné, linéaire ou ramifié, saturé ou insaturé, en $C_5$-$C_{50}$, ce radical pouvant être substitué par un ou plusieurs groupements hydroxyle, ce ou ces groupement(s) hydroxyle étant éventuellement estérifié(s) par un acide $R_{10}COOH$, $R_{10}$ étant un radical hydrocarboné, saturé ou insaturé, linéaire ou ramifié, éventuellement mono- ou polyhydroxylé, en $C_1$-$C_{35}$, le ou les groupements hydroxyle du radical $R_{10}$ pouvant être estérifié(s) par un acide gras saturé ou insaturé, linéaire ou ramifié, éventuellement mono- ou polyhydroxylé, en $C_1$-$C_{35}$,
  - soit un radical R"-(NR-CO)-R', dans lequel R désigne un atome d'hydrogène ou un radical hydrocarboné en $C_1$-$C_{20}$ mono- ou polyhydroxylé, préférentiellement monohydroxylé, R' et R" sont des radicaux hydrocarbonés dont la somme des atomes de carbone est comprise entre 9 et 30, R' étant un radical divalent,
  - soit un radical $R_{11}$-O-CO-$(CH_2)_p$, dans lequel $R_{11}$ désigne un radical hydrocarboné en $C_1$-$C_{20}$, p étant un nombre entier variant de 1 à 12 inclusivement ;

- $R_6$ désigne un atome d'hydrogène ou un radical $(glycosyle)_n$, $(galactosyle)_m$, sulfogalactosyle, phosphoryléthylamine ou phosphoryléthylammonium, dans lesquels n est un nombre entier variant de 1 à 4 inclusivement et m est

un nombre entier variant de 1 à 8 inclusivement ;

- $R_7$ désigne un atome d'hydrogène ou un radical hydrocarboné en $C_1$-$C_{33}$, saturé ou insaturé, hydroxylé ou non, le ou les radicaux hydroxyle pouvant être estérifié(s) par un acide minéral ou par un acide $R_{10}COOH$, $R_{10}$ ayant les mêmes significations que celles indiquées ci-dessus, le ou les radicaux hydroxyle pouvant être éthérifié(s) par un radical (glycosyle)$_n$, (galactosyle)$_m$, sulfogalactosyle, phosphoryléthylamine ou phosphoryléthylammonium, $R_7$ pouvant également être substitué par un ou plusieurs radicaux alkyle en $C_1$-$C_1$; $R_7$ désigne de préférence un radical $\alpha$-hydroxyalkyle en $C_{15}$-$C_{26}$ dont le groupement hydroxyle peut éventuellement être estérifié par un $\alpha$-hydroxyacide en $C_{16}$-$C_{30}$;

- $R_8$ désigne un atome d'hydrogène, un radical méthyle, éthyle, un radical hydrocarboné en $C_3$-$C_{50}$, saturé ou insaturé, linéaire ou ramifié, éventuellement hydroxylé ou un radical $-CH_2-CHOH-CH_2-O-R_{12}$ dans lequel $R_{12}$ désigne un radical hydrocarboné en $C_{10}$-$C_{26}$ ou un radical $R_{11}-O-CO-(CH_2)_p$, $R_{11}$ désignant un radical hydrocarboné en $C_1$-$C_{20}$ et p étant un nombre entier variant de 1 à 12 inclusivement ;

- $R_9$ désigne un atome d'hydrogène ou un radical hydrocarboné en $C_1$-$C_{30}$, saturé ou insaturé, linéaire ou ramifié, éventuellement mono- ou polyhydroxylé, le ou les radicaux hydroxyle pouvant être éthérifié(s) par un radical (glycosyle)$_n$, (galactosyle)$_m$, sulfogalactosyle, phosphoryléthylamine ou phosphoryléthyl-ammonium ;

sous réserve que lorsque $R_7$ et $R_9$ désignent un atome d'hydrogène ou lorsque $R_7$ désigne un atome d'hydrogène et $R_9$ désigne un radical méthyle, alors $R_8$ ne désigne pas un atome d'hydrogène, un radical méthyle ou éthyle.

Parmi les composés de formule (II) ci-dessus, on préfère les céramides et/ou glycocéramides dont les structures sont décrites par DOWNING dans Journal of Lipid Research, Vol. 35, page 2060-2068, 1994, ou ceux décrits dans la demande de brevet français FR-2 673 179, et dont les enseignements sont ici inclus à titre de référence.

Les composés de type céramide plus particulièrement préférés selon l'invention sont les composés de formule (II) dans lesquels $R_5$ désigne un radical alkyle saturé ou insaturé dérivé d'acides gras en $C_{14}$-$C_{22}$ éventuellement hydroxylé ; $R_6$ désigne un atome d'hydrogène ; et $R_7$ désigne un radical linéaire saturé en $C_{11}$-$C_{17}$ éventuellement hydroxylé, et de préférence en $C_{13}$-$C_{15}$.

De tels composés sont par exemple :

- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
- le N-palmitamidohexadécanediol,

et les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (II) pour lesquels $R_5$ désigne un radical alkyle saturé ou insaturé dérivé d'acides gras ; $R_6$ désigne un radical galactosyle ou sulfogalactosyle ; et $R_7$ désigne un radical hydrocarboné en $C_{12}$-$C_{22}$, saturé ou insaturé et de préférence un groupement $-CH=CH-(CH_2)_{12}-CH_3$.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (II) décrits dans les demandes de brevet EP-A-0 227 994 et WO 94 / 07 844.

De tels composés sont par exemple le QUESTAMIDE H, encore appelé bis-(N-hydroxyéthyl N-cétyl) malonamide et vendu par la société QUEST et le N-(2-hydroxyéthyl)-N-(3-cétyloxy-2-hydroxypropyl)amide d'acide cétylique.

On peut également utiliser le N-docosanoyl N-méthyl-D-glucamine tel que décrit dans la demande de brevet WO 94 / 24 097.

Parmi les bis-phénylalkylènediamines utilisables à titre de bases d'oxydation dans la composition tinctoriale mise en oeuvre au cours du procédé de l'invention, on peut notamment citer les composés répondant à la formule (III) suivante, et leurs sels d'addition avec un acide :

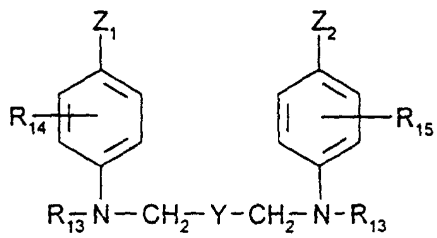

(III)

dans laquelle :

$Z_1$ et $Z_2$, identiques ou différents, représentent un radical hydroxyle ou $NHR_{16}$ dans lequel $R_{16}$ représente un atome d'hydrogène ou un radical alkyle en $C_1$-$C_4$, $R_{13}$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,

$R_{14}$ et $R_{15}$, identiques ou différents, représentent un atome d'hydrogène ou d'halogène ou un radical alkyle en $C_1$-$C_4$,

Y représente un radical pris dans le groupe constitué par les radicaux suivants : $-(CH_2)_n-$ ; $-(CH_2)_m-O-(CH_2)^-{}_m$ ; $-(CH_2)_m-CHOH-(CH_2)_m$ et

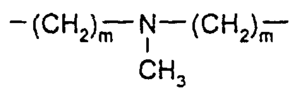

;

dans lesquels n est un nombre entier compris entre 0 et 8 inclusivement et m est un nombre entier compris entre 0 et 4 inclusivement.

Parmi les bis-phénylalkylènediamines de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxy-éthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans la composition tinctoriale mise en oeuvre au cours du procédé de l'invention, on peut notamment citer les composés répondant à la formule (IV) suivante, et leurs sels d'addition avec un acide :

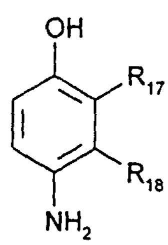

(IV)

dans laquelle :

$R_{17}$ représente un atome d'hydrogène, un radical alkyle en $C_1$-$C_4$, monohydroxyalkyle en $C_1$-$C_4$, alcoxy($C_1$-$C_4$) alkyle($C_1$-$C_4$), aminoalkyle en $C_1$-$C_4$ ou hydroxyalkyl($C_1$-$C_4$)aminoalkyle en $C_1$-$C_4$,

$R_{18}$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{17}$ et $R_{18}$ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les orthoaminophénols utilisables à titre de bases d'oxydation dans la composition tinctoriale mise en oeuvre au cours du procédé de l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans la composition tinctoriale mise en oeuvre au cours du procédé de l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.
Parmi dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.
Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, le 4,5-diamino 1-(4'-chlorobenzyl) pyrazole, et leurs sels d'addition avec un acide.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxy-éthyloxy paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Le ou les composés de type céramide représentent de préférence de 0,0001 % à 10 % en poids environ par rapport au poids total de la composition tinctoriale ou par rapport au poids total de la composition oxydante, et encore plus préférentiellement de 0,001 % à 5 % en poids environ.

La ou les bases d'oxydation représentent de préférence de 0,0001 à 20 % en poids environ du poids total de la composition tinctoriale, et encore plus préférentiellement de 0,005 à 10 % en poids environ.

Les compositions tinctoriales mises en oeuvre au cours du procédé de teinture de l'invention contiennent en outre généralement un ou plusieurs coupleurs choisis parmi les composés utilisés habituellement à ce titre en teinture d'oxydation, et parmi lesquels on peut citer les métadiphénols, les métaaminophénols, les métaphénylènediamines, les dérivés mono- ou polyhydroxylés du naphtalène, le sésamol et ses dérivés, les composés hétérocycliques tels que par exemple les coupleurs pyridiniques et les coupleurs indoliques, et leurs sels d'addition avec un acide.

Lorsqu'ils sont présents, le ou les coupleurs représentent de préférence de 0,0005 % à 20 % en poids environ du poids total de la composition tinctoriale, et encore plus préférentiellement de 0,01 % à 10 % en poids environ.

Les sels d'addition avec un acide utilisables dans le cadre des compositions tinctoriales mises en oeuvre au cours du procédé de l'invention (paraphénylènediamines de formule (I), bis-phénylalkylènediamines, para-aminophénols, ortho-aminophénols, bases hétérocycliques et coupleurs) sont notamment choisis parmi les chlorhydrates, les bromhydrates, les sulfates et les tartrates.

Le milieu approprié pour la teinture (ou support) des compositions tinctoriales mises en oeuvre au cours du procédé de teinture conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en

poids environ.

Le pH de la composition tinctoriale mise en oeuvre au cours du procédé de teinture conforme à l'invention est généralement compris entre 3 et 11,5, et encore plus préférentiellement entre 7 et 11. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante :

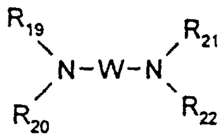

(V)

dans laquelle W est un reste propylène éventuellement substitué par un groupement hydroxyle ou un radical alkyle en $C_1$-$C_4$ ; $R_{19}$, $R_{20}$, $R_{21}$ et $R_{22}$, identiques ou différents, représentent un atome d'hydrogène, un radical alkyle en $C_1$-$C_4$ ou hydroxyalkyle en $C_1$-$C_4$.

La composition tinctoriale mise en oeuvre au cours du procédé de teinture conforme à l'invention peut encore contenir, en plus des colorants définis ci-dessus, des colorants directs, notamment pour modifier les nuances ou les enrichir en reflets.

La composition tinctoriale mise en oeuvre au cours du procédé de teinture de l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement au procédé de teinture conforme à l'invention ne soient pas, ou substantiellement pas, altérées par la ou les adjonctions envisagées.

La composition tinctoriale mise en oeuvre au cours du procédé de teinture de l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'agent oxydant présent dans la composition oxydante mise en oeuvre au cours du procédé de teinture de l'invention peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut en outre renfermer divers adjuvants utilisés classiquement dans les compositions pour la teinture des cheveux, et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

La composition tinctoriale mise en oeuvre au cours du procédé de l'invention et contenant au moins une base d'oxydation et au moins un composé de type céramide telle que décrite précédemment est nouvelle et constitue également un objet de l'invention.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité,

tel que les dispositifs décrits dans le brevet FR-2 586 913 au nom de la demanderesse.

Les exemples qui suivent sont destinés à illustrer l'invention sans pour autant en limiter la portée.

## EXEMPLES

### EXEMPLES 1 et 2 COMPARATIFS

On a réalisé les compositions tinctoriales suivantes (teneurs en grammes) :

| EXEMPLE | 1 (*) | 2 |
|---|---|---|
| Paraphénylènediamine | 0,108 | 0,108 |
| Métaaminophénol | 0,109 | 0,109 |
| N-oléoyldihydrosphingosine (céramide) | 0 | 2,5 |
| Support de teinture commun | (**) | (**) |
| Eau déminéralisée q.s.p. | 100 g | 100 g |

(*) : composition ne faisant pas partie de l'invention

(**) : Support de teinture commun :

- Alcool cétylique et stéarylique en mélange 50/50 — 18 g
- 2-octyl dodécanol — 3 g
- Alcool cétylstéarylique oxyéthyléné à 15 moles d'oxyde d'éthylène — 3 g
- Laurylsulfate d'ammonium à 30 % de matière active (M.A.) — 12 g
- Solution aqueuse à 60 % de M.A. d'un polymère cationique présentant le motif récurrent suivant :

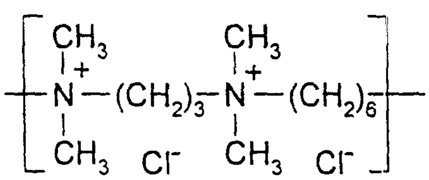

3 g M.A.

- Ammoniaque à 20 % de $NH_3$ — 12 g
- Thiolactate d'ammonium (à 50 % en équivalent d'acide thiolactique) — 0,8 g

Au moment de l'emploi, on a mélangé chaque composition tinctoriale avec une fois et demie son poids d'une

solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ainsi teintes ont ensuite subi un test de résistance aux intempéries. Ce test a pour but d'évaluer la dégradation de la coloration sous l'action simultanée de la lumière (Xénotest) et de l'eau douce.

Pour ce faire, les mèches de cheveux teintes ont été fixées sur un support (carton ou plastique). Ces supports ont été disposés sur des porte-échantillons que l'on a fait tourner autour d'une lampe Xénon pendant une durée de 64 heures sous un taux d'humidité relative de 60 % et à une température de 42,5 ± 2,5°C. Au cours de ces 64 heures, l'action de la lumière a été interrompue toutes les 12 heures, afin de faire subir aux mèches une pluie froide pendant une durée de 30 minutes.

La couleur des mèches a été évaluée dans le système MUNSELL, avant et après le test de résistance aux intempéries, au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur de chaque mèche avant et après le test de résistance à la lumière reflète la dégradation de la coloration due à l'action de la lumière et a été calculée en appliquant la formule de NICKERSON :

$$\Delta E = 0{,}4\ Co\Delta H + 6\Delta V + 3\ \Delta C$$

telle que décrite par exemple dans "Couleur, Industrie et Technique" ; pages 14-17 ; vol. n° 5 ; 1978.

Dans cette formule, ΔE représente la différence de couleur entre deux mèches, **ΔH**, **ΔV** et **ΔC** représentent la variation en valeur absolue des paramètres **H**, V et **C**, et **Co** représente la pureté de la mèche par rapport à laquelle on désire évaluer la différence de couleur (pureté de la mèche avant le test).

Les résultats sont donnés dans le tableau II ci-dessous :

| EXEMPLE | Couleur avant le test | Couleur après le test | Dégradation de la coloration | | | |
|---|---|---|---|---|---|---|
| | | | ΔH | ΔV | ΔC | ΔE |
| | | | | | | |
| **1 (*)** | 5,2 R 3,2 / 2,5 | 9,85 YR 5,5 / 2,7 | 14,65 | 2,3 | 0,2 | **29** |
| **2** | 5,4 R 3,7 / 2,7 | 9,05 YR 4,3 / 2,7 | 13,4 | 0,6 | 0 | **18,1** |

(*) : exemple ne faisant pas partie de l'invention

Ces résultats montrent que la composition 1 ne faisant pas partie de l'invention, car ne contenant pas de céramide, conduit sur cheveux à une coloration résistant beaucoup moins bien à l'action des intempéries que la coloration obtenue avec la composition 2 conforme à l'invention, c'est à dire contenant un céramide.

## Revendications

**1.** Procédé de teinture des fibres kératiniques et en particulier des fibres kératiniques humaines telles que les cheveux, caractérisé par le fait qu'on applique sur ces fibres :

- au moins une composition tinctoriale contenant, dans un milieu approprié pour la teinture, au moins une base d'oxydation choisie parmi les bis-phénylalkylènediamines, les para-aminophénols, les ortho-aminophénols, les bases hétérocycliques et les paraphénylènediamines de formule (I) suivante, et les sels d'addition avec un acide de ces composés :

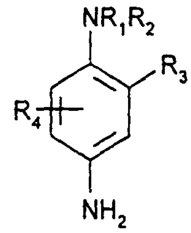 (I)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, phényle ou 4'-aminophényle,
$R_2$ représente un atome d'hydrogène, un radical alkyle en $C_1$-$C_4$, monohydroxyalkyle en $C_1$-$C_4$ ou polyhydroxyalkyle en $C_2$-$C_4$,
$R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$ ou hydroxyalcoxy en $C_1$-$C_4$,
$R_4$ représente un atome d'hydrogène ou un radical alkyle en $C_1$-$C_4$;

- la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée ;

ladite composition tinctoriale et/ou ladite composition oxydante contenant au moins un composé de type céramide.

**2.** Procédé selon la revendication 1, caractérisé par le fait qu'on mélange, au moment de l'emploi, ladite composition tinctoriale avec ladite composition oxydante contenant, dans un milieu approprié pour la teinture, au moins un agent oxydant présent en une quantité suffisante pour développer une coloration.

**3.** Procédé selon la revendication 1 ou 2, caractérisé par le fait que le ou les composés de type céramide sont choisis parmi les molécules naturelles ou synthétiques répondant à la formule (II) suivante :

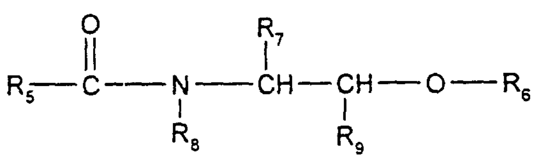 (II)

dans laquelle :

- $R_5$ désigne :

  - soit un radical hydrocarboné, linéaire ou ramifié, saturé ou insaturé, en $C_5$-$C_{50}$, ce radical pouvant être substitué par un ou plusieurs groupements hydroxyle, ce ou ces groupement(s) hydroxyle étant éventuellement estérifié(s) par un acide $R_{10}COOH$, $R_{10}$ étant un radical hydrocarboné, saturé ou insaturé, linéaire ou ramifié, éventuellement mono- ou polyhydroxylé, en $C_1$-$C_{35}$, le ou les groupements hydroxyle du radical $R_{10}$ pouvant être estérifié(s) par un acide gras saturé ou insaturé, linéaire ou ramifié, éventuellement mono- ou polyhydroxylé, en $C_1$-$C_{35}$,

  - soit un radical R"-(NR-CO)-R' dans lequel R désigne un atome d'hydrogène ou un radical hydrocarboné en $C_1$-$C_{20}$ mono- ou polyhydroxylé, préférentiellement monohydroxylé, R' et R" sont des radicaux hydrocarbonés dont la somme des atomes de carbone est comprise entre 9 et 30, R' étant un radical divalent,

  - soit un radical $R_{11}$-O-CO-$(CH_2)_p$, dans lequel $R_{11}$ désigne un radical hydrocarboné en $C_1$-$C_{20}$, p étant un nombre entier variant de 1 à 12 inclusivement ;

- $R_6$ désigne un atome d'hydrogène ou un radical (glycosyle)$_n$, (galactosyle)$_m$, sulfogalactosyle, phosphoryléthylamine ou phosphoryléthylammonium, dans lesquels n est un nombre entier variant de 1 à 4 inclusivement et m est un nombre entier variant de 1 à 8 inclusivement ;

- $R_7$ désigne un atome d'hydrogène ou un radical hydrocarboné en $C_1$-$C_{33}$, saturé ou insaturé, hydroxylé ou non, le ou les radicaux hydroxyle pouvant être estérifié(s) par un acide minéral ou par un acide $R_{10}COOH$, $R_{10}$ ayant les mêmes significations que celles indiquées ci-dessus, le ou les radicaux hydroxyle pouvant être éthérifié(s) par un radical (glycosyle)$_n$, (galactosyle)$_m$, sulfogalactosyle, phosphoryléthylamine ou phosphoryléthylammonium, $R_7$ pouvant également être substitué par un ou plusieurs radicaux alkyle en $C_1$-$C_{14}$ ; $R_7$ désigne de préférence un radical $\alpha$-hydroxyalkyle en $C_{15}$-$C_{26}$ dont le groupement hydroxyle peut éventuellement être estérifié par un $\alpha$-hydroxyacide en $C_{16}$-$C_{30}$ ;

- $R_8$ désigne un atome d'hydrogène, un radical méthyle, éthyle, un radical hydrocarboné en $C_3$-$C_{50}$, saturé ou insaturé, linéaire ou ramifié, éventuellement hydroxylé ou un radical $-CH_2-CHOH-CH_2-O-R_{12}$ dans lequel $R_{12}$ désigne un radical hydrocarboné en $C_{10}$-$C_{26}$ ou un radical $R_{11}-O-CO-(CH_2)_p$, $R_{11}$ désignant un radical hydrocarboné en $C_1$-$C_{20}$ et p étant un nombre entier variant de 1 à 12 inclusivement ;

- $R_9$ désigne un atome d'hydrogène ou un radical hydrocarboné en $C_1$-$C_{30}$, saturé ou insaturé, linéaire ou ramifié, éventuellement mono- ou polyhydroxylé, le ou les radicaux hydroxyle pouvant être éthérifié(s) par un radical (glycosyle)$_n$, (galactosyle)$_m$, sulfogalactosyle, phosphoryléthylamine ou phosphoryléthylammonium ;

sous réserve que lorsque $R_7$ et $R_9$ désignent un atome d'hydrogène ou lorsque $R_7$ désigne un atome d'hydrogène et $R_9$ désigne un radical méthyle, alors $R_8$ ne désigne pas un atome d'hydrogène, un radical méthyle ou éthyle.

**4.** Procédé selon la revendication précédente, caractérisé par le fait que les composés de type céramide sont choisis parmi les composés de formule (II) dans lesquels $R_5$ désigne un radical alkyle saturé ou insaturé dérivé d'acides gras en $C_{14}$-$C_{22}$ éventuellement hydroxyle ; $R_6$ désigne un atome d'hydrogène ; et $R_7$ désigne un radical linéaire saturé en $C_{11}$-$C_{17}$ éventuellement hydroxylé, et de préférence en $C_{13}$-$C_{15}$.

**5.** Procédé selon la revendication précédente, caractérisé par le fait que les céramides sont choisis parmi :

- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
- le N-palmitamidohexadécanediol, et les mélanges de ces composés.

**6.** Procédé selon la revendication 3, caractérisé par le fait que les céramides sont choisis parmi les composés de formule (II) dans lesquels

- $R_5$ désigne un radical alkyle saturé ou insaturé dérivé d'acides gras ;
- $R_6$ désigne un radical galactosyle ou sulfogalactosyle ;
- et $R_7$ désigne un radical hydrocarboné en $C_{12}$-$C_{22}$, saturé ou insaturé et de préférence un groupement $-CH=CH-(CH_2)_{12}-CH_3$.

**7.** Procédé selon l'une quelconque des revendications précédentes, caractérisé par le fait que les bis-phénylalkylènediamines sont choisies parmi les composés de formule (III) suivante, et leurs sels d'addition avec un acide :

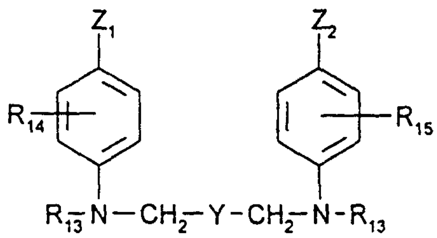

(III)

dans laquelle :

$Z_1$ et $Z_2$, identiques ou différents, représentent un radical hydroxyle ou $NHR_{16}$ dans lequel $R_{16}$ représente un atome d'hydrogène ou un radical alkyle en $C_1$-$C_4$, $R_{13}$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,

$R_{14}$ et $R_{15}$, identiques ou différents, représentent un atome d'hydrogène ou d'halogène ou un radical alkyle en $C_1$-$C_4$,

Y représente un radical pris dans le groupe constitué par les radicaux suivants : $-(CH_2)_n^-$ ; $-(CH_2)_m$-o-$(CH_2)^-_m$ ; $-(CH_2)_m$-CHOH-$(CH_2)^-_m$ et

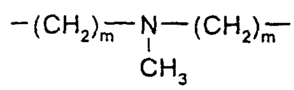

;

dans lesquels n est un nombre entier compris entre 0 et 8 inclusivement et m est un nombre entier compris entre 0 et 4 inclusivement.

8. Procédé selon la revendication 7, caractérisé par le fait que les bis-phénylalkylènediamines de formules (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N, N'-bis-(β-hydroxyéthyl) N,N'-bis-(4-aminophényl) tétraméthylènediamine, la N,N'-bis-(4-méthylamino-phényl) tétraméthylènediamine, la N,N'-bis-(éthyl) N,N'-bis-(4'-amino, 3'-méthylphényl) éthylènediamine, et leurs sels d'addition avec un acide.

9. Procédé selon l'une quelconque des revendications précédentes, caractérisé par le fait que les para-aminophénols sont choisis parmi les composés répondant à la formule (IV) suivante, et leurs sels d'addition avec un acide :

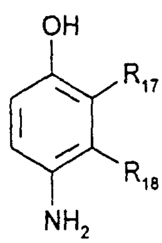

(IV)

dans laquelle :

$R_{17}$ représente un atome d'hydrogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,

$R_{18}$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{17}$ et $R_{18}$ représente un atome d'hydrogène.

**10.** Procédé selon la revendication 9, caractérisé par le fait que les para-aminophénols de formule (IV) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

**11.** Procédé selon l'une quelconque des revendications précédentes, caractérisé par le fait que les orthoaminophénols sont choisis parmi 2-amino phénol, le 2-amino 5-méthyl phénol, le 2-amino 6-méthyl phénol, le 5-acétamido 2-amino phénol, et leurs sels d'addition avec un acide.

**12.** Procédé selon l'une quelconque des revendications précédentes, caractérisé par le fait que les bases hétérocycliques sont choisies parmi les dérivés pyridiniques, les dérivés pyrimidiniques, les dérivés pyrazoliques, et leurs sels d'addition avec un acide.

**13.** Procédé selon la revendication 12, caractérisé par le fait que les bases d'oxydation hétérocycliques sont choisies parmi la 2,4,5,6-tétra-aminopyrimidine, la 2,5-diaminopyridine, le 4,5-diamino 1-méthyl pyrazole, le 3,4-diamino pyrazole, le 4,5-diamino 1-(4'-chlorobenzyl) pyrazole, la 4-hydroxy 2,5,6-triaminopyrimidine, et leurs sels d'addition avec un acide.

**14.** Procédé selon l'une quelconque des revendications précédentes, caractérisé par le fait que les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la-4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, et leurs sels d'addition avec un acide.

**15.** Procédé selon la revendication 14, caractérisé par le fait que les paraphénylènediamines de formule (I) sont choisies parmi la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

**16.** Procédé selon l'une quelconque des revendications précédentes, caractérisé par le fait que le ou les composés de type céramide représentent de 0,0001 % à 10 % en poids par rapport au poids total de la composition tinctoriale ou par rapport au poids total de la composition oxydante.

**17.** Procédé selon la revendication 16, caractérisé par le fait que le ou les composés de type céramide représentent de 0,001 % à 5 % en poids par rapport au poids total de la composition tinctoriale ou par rapport au poids total de la composition oxydante.

**18.** Procédé selon l'une quelconque des revendications précédentes, caractérisé par le fait que la ou les bases d'oxydation représentent de 0,0001 à 20 % en poids du poids total de la composition tinctoriale.

**19.** Procédé selon la revendication 17, caractérisé par le fait que la ou les bases d'oxydation représentent de 0,005 à 10 % en poids du poids total de la composition tinctoriale.

**20.** Procédé selon l'une quelconque des revendications précédentes, caractérisé par le fait que la composition tinctoriale contient en outre un ou plusieurs coupleurs choisis parmi les métadiphénols, les métaaminophénols, les métaphénylènediamines, les dérivés mono- ou polyhydroxylés du naphtalène, le sésamol et ses dérivés, les composés hétérocycliques tels que par exemple les coupleurs pyridiniques et les coupleurs indoliques, et leurs sels d'addition avec un acide.

**21.** Procédé selon la revendication 20, caractérisé par le fait que le ou les coupleurs représentent de 0,0005 % à 20 % en poids du poids total de la composition tinctoriale.

**22.** Procédé selon la revendication 21, caractérisé par le fait que le ou les coupleurs représentent de 0,01 % à 10 % en poids du poids total de la composition tinctoriale.

**23.** Procédé selon l'une quelconque des revendications précédentes, caractérisé par le fait que les sels d'addition avec un acide sont choisis parmi les chlorhydrates, les bromhydrates, les sulfates et les tartrates.

**24.** Procédé selon l'une quelconque des revendications précédentes, caractérisé par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**25.** Procédé selon l'une quelconque des revendications précédentes, caractérisé par le fait la composition tinctoriale présente un pH compris entre 3 et 11,5.

**26.** Procédé selon l'une quelconque des revendications précédentes, caractérisé par le fait que l'agent oxydant est choisi parmi le peroxyde d'hydrogène, le peroxyde d'urée, les bromates de métaux alcalins, les persels tels que les perborates et persulfates.

**27.** Composition pour la teinture d'oxydation des fibres kératiniques et en particulier des fibres kératiniques humaines telles que les cheveux, caractérisée par le fait qu'elle comprend, clans un milieu approprié pour la teinture :

- au moins une base d'oxydation choisie parmi les bis-phénylalkylènediamines, les para-aminophénols, les ortho-aminophénols, les bases hétérocycliques et les paraphénylènediamines de formule (I) suivante, et les sels d'addition avec un acide de ces composés:

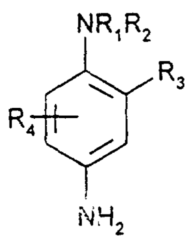

(I)

dans laquelle:

$R_1$ représente un atome d'hydrogène, un radical alkyle en $C_1$-$C_4$, monohydroxyalkyle en $C_1$-$C_4$, polyhydroxyalkyle en $C_2$-$C_4$, phényle ou 4'-aminophényle,
$R_2$ représente un atome d'hydrogène, un radical alkyle en $C_1$-$C_4$, monohydroxyalkyle en $C_1$-$C_4$ ou polyhydroxyalkyle en $C_2$-$C_4$,
$R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$ ou hydroxyalcoxy en $C_1$-$C_4$,
$R_4$ représente un atome d'hydrogène ou un radical alkyle en $C_1$-$C_4$ ;

- et au moins un composé de type céramide.

**28.** Dispositif à plusieurs compartiments, ou "kit" de teinture à plusieurs compartiments, dont un premier compartiment renferme une composition tinctoriale contenant, dans un milieu approprié pour la teinture, au moins une base d'oxydation choisie parmi les bis-phénylalkylènediamines, les para-aminophénols, les ortho-aminophénols, les bases hétérocycliques et les paraphénylènediamines de formule (I) suivante, et les sels d'addition avec un acide de ces composés :

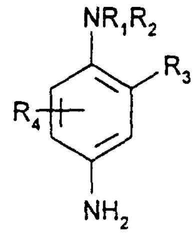

(I)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, phényle ou 4'-aminophényle,
$R_2$ représente un atome d'hydrogène, un radical alkyle en $C_1$-$C_4$, monohydroxyalkyle en $C_1$-$C_4$ ou polyhydroxyalkyle en $C_2$-$C_4$,
$R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$ ou hydroxyalcoxy en $C_1$-$C_4$,
$R_4$ représente un atome d'hydrogène ou un radical alkyle en $C_1$-$C_4$;

et un second compartiment renferme une composition oxydante ;
ladite composition tinctoriale et/ou ladite composition oxydante contenant au moins un composé de type céramide.

## Patentansprüche

1. Verfahren zum Färben von Keratinfasern und insbesondere menschlichen Keratinfasern, wie dem Haar, **dadurch gekennzeichnet**, daß auf die Keratinfasern aufgetragen wird:

- mindestens eine Färbemittelzusammensetzung, die in einem zum Färben geeigneten Medium mindestens eine Oxidationsbase enthält, die unter den Bisphenylalkylendiaminen, p-Aminophenolen, o-Aminophenolen, heterocyclischen Basen und p-Phenylendiaminen der folgenden Formel (I) und den Additionssalzen dieser Verbindungen mit einer Säure ausgewählt ist:

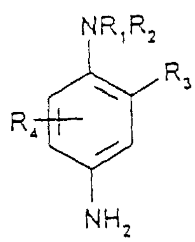

(I),

worin bedeuten:

$R_1$ Wasserstoff, $C_{1-4}$-Alkyl, $C_{1-4}$-Monohydroxyalkyl, $C_{2-4}$-Polyhydroxyalkyl, Phenyl oder 4'-Aminophenyl;
$R_2$ Wasserstoff, $C_{1-4}$-Alkyl, $C_{1-4}$-Monohydroxyalkyl oder $C_{2-4}$-Polyhydroxyalkyl;
$R_3$ Wasserstoff, Halogen, wie Chlor, Brom, Iod oder Fluor, $C_{1-4}$-Alkyl, $C_{1-4}$-Monohydroxyalkyl oder $C_{1-4}$-Hydroxyalkoxy;
R4 Wasserstoff oder $C_{1-4}$-Alkyl.

- wobei die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird;

wobei die Färbemittelzusammensetzung und/oder die oxidierende Zusammensetzung mindestens eine Verbindung vom Ceramidtyp enthält.

**2.** Verfahren nach Anspruch 1, **dadurch gekennzeichnet**, daß die Färbemittelzusammensetzung bei der Anwendung mit der oxidierenden Zusammensetzung vermischt wird, die in einem zum Färben geeigneten Medium mindestens ein Oxidationsmittel enthält, das in einer zur Bildung einer Färbung ausreichenden Menge vorliegt.

**3.** Verfahren nach Anspruch 1 oder 2, **dadurch gekennzeichnet**, daß die Verbindung(en) vom Ceramidtyp unter den natürlichen oder synthetischen Molekülen der folgenden Formel (II) ausgewählt sind:

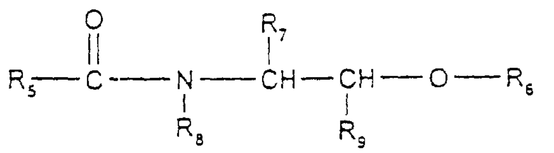

(II),

worin bedeuten:

- $R_5$ entweder eine geradkettige oder verzweigte, gesättigte oder ungesättigte $C_{5-50}$-Kohlenwasserstoffgruppe, wobei die Gruppe mit einer oder mehreren Hydroxygruppen substituiert sein kann, die gegebenenfalls mit einer Säure $R_{10}COOH$ verestert sein können, wobei $R_{10}$ eine gesättigte oder ungesättigte, geradkettige oder verzweigte $C_{1-35}$-Kohlenwasserstoffgruppe ist, die gegebenenfalls eine oder mehrere Hydroxygruppen aufweist, wobei die Hydroxygruppe(n) der Gruppe $R_{10}$ mit einer gesättigten oder ungesättigten, geradkettigen oder verzweigten, gegebenenfalls ein- oder mehrfach hydroxylierten $C_{1-35}$-Fettsäure verestert sein kann (können);
  oder eine Gruppe R''-(NR-CO)-R', wobei R Wasserstoff oder eine $C_{1-20}$-Kohlenwasserstoffgruppe mit einer oder mehreren Hydroxygruppen und vorzugsweise einer Hydroxygruppe bedeutet und R' und R'' Kohlenwasserstoffgruppen sind, deren Summe an Kohlenstoffatomen im Bereich von 9 bis 30 liegt, wobei R' eine zweiwertige Gruppe ist,
  oder eine Gruppe $R_{11}$-O-CO-$(CH_2)_p$, wobei $R_{11}$ eine $C_{1-20}$-Kohlenwasserstoffgruppe ist und p eine ganze Zahl im Bereich von 1 bis 12 bedeutet;

- $R_6$ Wasserstoff oder eine Gruppe $(Glycosyl)_n$, $(Galactosyl)_m$ oder Sulfogalactosyl, Phosphorylethylamin oder Phosphorylethylammonium, wobei n eine ganze Zahl im Bereich von 1 bis 4 und m eine ganze Zahl im Bereich von 1 bis 8 ist;

- $R_7$ Wasserstoff oder eine gesättigte oder ungesättigte, gegebenenfalls hydroxylierte $C_{1-33}$-Kohlenwasserstoffgruppe, wobei die Hydroxygruppe(n) mit: einer anorganischen Säure oder einer Säure $R_{10}COOH$, wobei $R_{10}$ die oben angegebenen Bedeutungen aufweist, verestert oder mit einer $(Glycosyl)_n$-Gruppe, $(Galactosyl)_m$-Gruppe, Sulfogalactosylgruppe, Phosphorylethylamingruppe oder einer Phosphorylethylammoniumgruppe verethert sein kann (können), und wobei die Gruppe $R_7$ mit einer oder mehreren $C_{1-14}$-Alkylgruppen substituiert sein kann; $R_7$ bedeutet vorzugsweise eine $\alpha$-Hydroxyalkylgruppe mit 15 bis 26 Kohlenstoffatomen, wobei die Hydroxygruppe gegebenenfalls mit einer $\alpha$-Hydroxysäure mit 16 bis 30 Kohlenstoffatomen verestert sein kann;

- $R_8$ Wasserstoff, Methyl, Ethyl, eine gesättigte oder ungesättigte, geradkettige oder verzweigte, gegebenenfalls hydroxylierte $C_{3-50}$-Kohlenwasserstoffgruppe, eine Gruppe $-CH_2$-CHOH-$CH_2$-O-$R_{12}$, wobei $R_{12}$ eine $C_{10-26}$-Kohlenwasserstoffgruppe bedeutet, oder eine Gruppe $R_{11}$-O-CO-$(CH_2)_p$, wobei $R_{11}$ eine $C_{1-20}$-Kohlenwasserstoffgruppe bedeutet und p eine ganze Zahl im Bereich von 1 bis 12 ist;

- $R_9$ Wasserstoff oder eine gesättigte oder ungesättigte, geradkettige oder verzweigte, gegebenenfalls ein- oder mehrfach hydroxylierte $C_{1-30}$-Kohlenwasserstoffgruppe, wobei die Hydroxygruppe(n) mit einer $(Glycosyl)_n$-Gruppe, $(Galactosyl)_m$-Gruppe, Sulfogalactosylgruppe, Phosphorylethylamingruppe und einer Phosphorylethylammoniumgruppe verethert sein kann (können);

mit der Maßgabe, daß $R_8$ nicht Wasserstoff, Methyl oder Ethyl bedeutet, wenn $R_7$ und $R_9$ Wasserstoff bedeuten oder wenn $R_7$ Wasserstoff und $R_9$ Methyl bedeutet.

**4.** Verfahren nach dem vorhergehenden Anspruch, **dadurch gekennzeichnet**, daß die Verbindungen vom Ceramidtyp unter den Verbindungen der Formel (II) ausgewählt sind, worin $R_5$ eine gesättigte oder ungesättigte, von $C_{14-22}$-

Fettsäuren abgeleitete, gegebenenfalls hydroxylierte Alkylgruppe, $R_6$ Wasserstoff und $R_7$ eine gesättigte, geradkettige, gegebenenfalls hydroxylierte $C_{11-17}$-Gruppe und vorzugsweise $C_{13-15}$-Gruppe bedeutet.

**5.** Verfahren nach dem vorhergehenden Anspruch, **dadurch gekennzeichnet**, daß die Ceramide ausgewählt sind unter:

- N-Linoleoyl-dihydrosphingosin,
- N-Oleoyl-dihydrosphingosin,
- N-Palmitoyl-dihydrosphingosin,
- N-Stearoyl-dihydrosphingosin,
- N-Behenoyl-dihydrosphingosin,
- N-2-Hydroxypalmitoyl-dihydrosphingosin,
- N-Stearoylphytosphingosin,
- N-Palmitamido-hexadecandiol, und

den Gemischen dieser Verbindungen.

**6.** Verfahren nach Anspruch 3, **dadurch gekennzeichnet**, daß die Ceramide unter den Verbindungen der Formel (II) ausgewählt sind, worin:

- $R_5$ eine gesättigte oder ungesättigte, von Fettsäuren abgeleitete Alkylgruppe,
- $R_6$ eine Galactosyl- oder Sulfogalactosylgruppe, und
- $R_7$ eine gesättigte oder ungesättigte $C_{12-22}$-Kohlenwasserstoffgruppe und vorzugsweise die Gruppe $-CH=CH-(CH_2)_{12}-CH_3$ bedeutet.

**7.** Verfahren nach einem der vorhergehenden Ansprüche, **dadurch gekennzeichnet**, daß die Bisphenylalkylendiamine unter den Verbindungen der folgenden Formel (III) und den Additionssalze dieser Verbindungen mit einer Säure ausgewählt sind:

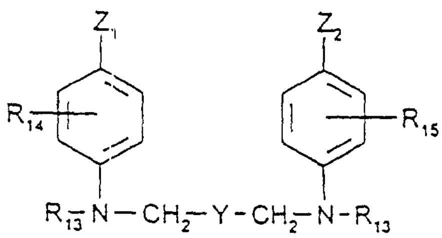

(III)

worin bedeuten:

$Z_1$ und $Z_2$, die identisch oder voneinander verschieden sind, Hydroxy oder $NHR_{16}$, wobei $R_{16}$ Wasserstoff oder $C_{1-4}$-Alkyl bedeutet,
$R_{13}$ Wasserstoff, $C_{1-4}$-Alkyl, $C_{1-4}$-Monohydroxyalkyl, $C_{2-4}$-Polyhydroxyalkyl oder $C_{1-4}$-Aminoalkyl, wobei die Aminoguppe substituiert sein kann,
$R_{14}$ und $K_{15}$, die identisch oder voneinander verschieden sind, ein Wasserstoff- oder Halogenatom oder $C_{1-4}$-Alkyl,
Y eine Gruppe, die unter den folgenden Gruppen ausgewählt ist: $-(CH_2)_n-$; $-(CH_2)_m-O-(CH_2)_m-$; $-(CH_2)_m-CHOH-(CH_2)_m-$ und

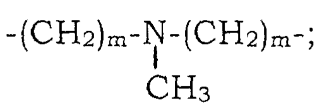

wobei n Null oder eine ganze Zahl von 1 bis 8 und m Null oder eine ganze Zahl von 1 bis 4 bedeutet.

**8.** Verfahren nach Anspruch 7, **dadurch gekennzeichnet**, daß die Bisphenylalkylendiamine der Formel (III) ausgewählt sind unter: N,N'-Bis(β-hydroxyethyl)-N,N'-bis(4'-aminophenyl)-1,3-diamino-2-propanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis-(ethyl)-N,N'-bis(4'-amino-3'-methylphenyl)-ethylendiamin und den Additionssalzen dieser Verbindungen mit einer Säure.

**9.** Verfahren nach einem der vorhergehenden Ansprüche, **dadurch gekennzeichnet**, daß die p-Aminophenole unter den Verbindungen der folgenden Formel (IV) und den Additionssalzen dieser Verbindungen mit einer Säure ausgewählt sind:

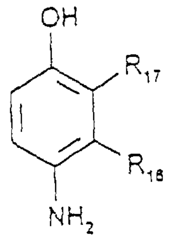

(IV)

worin bedeuten:

$R_{17}$ Wasserstoff, $C_{1-4}$-Alkyl, $C_{1-4}$-Monohydroxyalkyl, $C_{1-4}$-Alkoxy-$C_{1-4}$-alkyl, $C_{1-4}$-Aminoalkyl oder $C_{1-4}$-Hydroxyalkyl-$C_{1-4}$-aminoalkyl;
$R_{18}$ Wasserstoff, Fluor, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxy-$C_{1-4}$-alkyl;

mit der Maßgabe, daß mindestens eine der Gruppen $R_{17}$ oder $R_{18}$ Wasserstoff bedeutet.

**10.** Verfahren nach Anspruch 9, **dadurch gekennzeichnet**, daß die p-Aminophenole der Formel (IV) ausgewählt sind unter: p-Amino-phenol, 4-Amino-3-methylphenol, 4-Amino-3-fluorphenol, 4-Amino-3-hydroxy-methylphenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethylaminomethyl)-phenol und den Additionssalzen dieser Verbindungen mit einer Säure.

**11.** Verfahren nach einem der vorhergehenden Ansprüche, **dadurch gekennzeichnet**, daß die o-Aminophenole unter 2-Aminophenol, 2-Amino-5-methylphenol, 2-Amino-6-methylphenol, 5-Acetamido-2-aminophenol und den Additionssalzen dieser Verbindungen mit einer Säure ausgewählt sind.

**12.** Verfahren nach einem der vorhergehenden Ansprüche, **dadurch gekennzeichnet**, daß die heterocyclischen Basen unter den Pyridinderivaten, Pyrimidinderivaten, Pyrazolderivaten und den Additionssalzen dieser Verbindungen mit einer Säure ausgewählt sind.

**13.** Verfahren nach Anspruch 12, **dadurch gekennzeichnet**, daß die heterocyclischen Basen unter 2,4,5,6-Tetraaminopyrimidin, 2,5-Diaminopyridin, 4,5-Diamino-1-methylpyrazol, 3,4-Diaminopyrazol, 4,5-Diamino-1-(4'-chlorbenzyl)-pyrazol, 4-Hydroxy-2,5,6-triamino-pyrimidin und den Additionssalzen dieser Verbindungen mit einer Säure ausgewählt sind.

**14.** Verfahren nach einem der vorhergehenden Ansprüche, **dadurch gekennzeichnet**, daß die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-Amino- N,N-bis(β-hydroxyethyl)-3-methyl-anilin, 4-Amino-3-chlor-N,N-bis(ß-hydroxyethyl)-anilin, 2-β-Hydroxyethyl-p-phenylendiamin, 2-Fluor-p-phenylendiamin, 2-Isopropyl-p-phenylendiamin, N-(β-Hydroxy-propyl)-p-phenylendiamin, 2-Hydroxymethyl-p-phenylendiamin, N,N-Dimethyl-3-methyl-p-phenylendiamin, N,N-(Ethyl, β-hydroxy-ethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin und deren Additionssalzen mit einer Säure ausgewählt sind.

**15.** Verfahren nach Anspruch 14, **dadurch gekennzeichnet**, daß die p-Phenylendiamine der Formel (I) unter p-Phenylendiamin, p-Toluylen-diamin, 2-Isopropyl-p-phenylendiamin, 2-β-Hydroxyethyl-p-phenylendiamin, 2-β-Hydroxyethyloxy-p-phenylendiamin, 2,6-Dimethyl-p-phenylen-diamin, 2,6-Diethyl-p-phenylendiamin, 2,3-Dimethyl-p-phenylendiamin, N,N-Bis(β-hydroxyethyl)-p-phenylen-diamin, 2-Chlor-p-phenylendiamin und deren Additionssalzen mit einer Säure ausgewählt sind.

**16.** Verfahren nach einem der vorhergehenden Ansprüche, **dadurch gekennzeichnet**, daß die Verbindung(en) vom Ceramidtyp 0,0001 bis 10 Gew.-%, bezogen auf das Gesamtgewicht der Färbemittelzusammensetzung oder das Gesamtgewicht der oxidierenden Zusammensetzung, ausmachen.

**17.** Verfahren nach Anspruch 16, **dadurch gekennzeichnet**, daß die Verbindung(en) vom Ceramidtyp 0,001 bis 5 Gew.-%, bezogen auf das Gesamtgewicht der Färbemittelzusammensetzung oder das Gesamtgewicht der oxidierenden Zusammensetzung, ausmachen.

**18.** Verfahren nach einem der vorhergehenden Ansprüche, **dadurch gekennzeichnet**, daß die Oxidationsbase(n) 0,0001 bis 20 Gew.-%, bezogen auf das Gesamtgewicht der Färbemittelzusammensetzung, ausmachen.

**19.** Verfahren nach Anspruch 17, **dadurch gekennzeichnet**, daß die Oxidationsbase(n) 0,005 bis 10 Gew.-% des Gesamtgesichts der Färbemittelzusammensetzung ausmachen.

**20.** Verfahren nach einem der vorhergehenden Ansprüche, **dadurch gekennzeichnet**, daß die Färbemittelzusammensetzung ferner einen oder mehrere Kuppler enthält, die unter den m-Dihydroxybenzolen, m-Aminophenolen, m-Phenylendiaminen, mono- oder polyhydroxylierten Naphthalinderivaten, Sesamol und seinen Derivaten und den heterocyclischen Verbindungen, wie beispielsweise Pyridinkupplern und Indolkupplern, und den Additionssalzen dieser Verbindungen mit einer Säure ausgewählt sind.

**21.** Verfahren nach Anspruch 20, **dadurch gekennzeichnet**, daß der oder die Kuppler 0,0005 bis 20 Gew.-% des Gesamtgewichts der Färbemittelzusammensetzung ausmachen.

**22.** Verfahren nach Anspruch 21, **dadurch gekennzeichnet**, daß der oder die Kuppler 0,01 bis 10 Gew.-% des Gesamtgewichts der Färbemittelzusammensetzung ausmachen.

**23.** Verfahren nach einem der vorhergehenden Ansprüche, **dadurch gekennzeichnet**, daß die Additionssalze mit einer Säure unter den Hydrochloriden, Hydrobromiden, Sulfaten und Tartraten ausgewählt sind.

**24.** Verfahren nach einem der vorhergehenden Ansprüche, **dadurch gekennzeichnet**, daß das zum Färben geeignete Medium (oder der Träger) aus Wasser oder einem Gemisch von Wasser und mindestens einem organischen Lösungsmittel besteht, das unter den niederen $C_{1-4}$-Alkanolen, Glycerin, den Glykolen und Glykolethern, den aromatischen Alkoholen, analogen Produkten und deren Gemischen ausgewählt ist.

**25.** Verfahren nach einem der vorhergehenden Ansprüche, **dadurch gekennzeichnet**, daß die Zusammensetzung einen pH-Wert im Bereich von 3 bis 11,5 aufweist.

**26.** Verfahren nach einem der vorhergehenden Ansprüche, **dadurch gekennzeichnet**, daß das Oxidationsmittel unter Wasserstoffperoxid, Harnstoffperoxid, Alkalimetallbromaten und die Salzen von Persäuren, wie Perboraten und Persulfaten, ausgewählt ist.

**27.** Zusammensetzung zum oxidativen Färben von Keratinfasern und insbesondere menschlichen Keratinfasern, wie dem Haar, **dadurch gekennzeichnet**, daß sie in einem zum Färben geeigneten Medium enthält:

- mindestens eine Oxidationsbase, die unter den Bisphenylalkylendiaminen, p-Aminophenolen, o-Aminophenolen, heterocyclischen Basen und p-Phenylendiaminen der folgenden Formel (I) und den Additionssalzen dieser Verbindungen mit einer Säure ausgewählt ist:

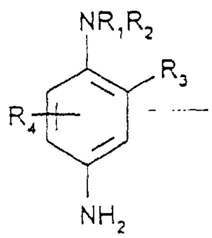

(I),

worin bedeuten:

$R_1$ Wasserstoff, $C_{1-4}$-Alkyl, $C_{1-4}$-Monohydroxyalkyl, $C_{2-4}$-Polyhydroxyalkyl, Phenyl oder 4'-Aminophenyl;
$R_2$ Wasserstoff, $C_{1-4}$-Alkyl, $C_{1-4}$-Monohydroxyalkyl oder $C_{2-4}$-Polyhydroxyalkyl;
$R_3$ Wasserstoff, Halogen, wie Chlor, Brom, Iod oder Fluor, $C_{1-4}$-Alkyl, $C_{1-4}$-Monohydroxyalkyl oder $C_{1-4}$-Hydroxyalkoxy;
R4 Wasserstoff oder $C_{1-4}$-Alkyl, und

- mindestens eine Verbindung vom Ceramidtyp

**28.** Vorrichtung mit mehreren Abteilungen oder "Kit" zum Färben mit mehreren Abteilungen, wobei eine erste Abteilung eine Färbemittelzusammensetzung, die in einem zum Färben geeigneten Medium mindestens eine Oxidationsbase enthält, die unter den Bisphenylalkylendiaminen, p-Aminophenolen, o-Aminophenolen, heterocyclischen Basen und den p-Phenylendiaminen der folgenden Formel (I) und den Additionssalzen dieser Verbindungen mit einer Säure ausgewählt ist:

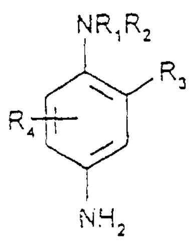

(I),

worin bedeuten:

$R_1$ Wasserstoff, $C_{1-4}$-Alkyl, $C_{1-4}$-Monohydroxyalkyl, $C_{2-4}$-Polyhydroxyalkyl, Phenyl oder 4'-Aminophenyl;
$R_2$ Wasserstoff, $C_{1-4}$-Alkyl, $C_{1-4}$-Monohydroxyalkyl oder $C_{2-4}$-Polyhydroxyalkyl;
$R_3$ Wasserstoff, Halogen, wie Chlor, Brom, Iod oder Fluor, $C_{1-4}$-Alkyl, $C_{1-4}$-Monohydroxyalkyl oder $C_{1-4}$-Hydroxyalkoxy;
$R_4$ Wasserstoff oder $C_{1-4}$-Alkyl;

und eine zweite Abteilung eine oxidierende Zusammensetzung enthält,
wobei die Färbemittelzusammensetzung und/oder die oxidierende Zusammensetzung mindestens eine Verbindung vom Ceramidtyp enthalten.

## Claims

**1.** Process for dyeing keratin fibres, and in particular human keratin fibres such as the hair, **characterized in that** the following are applied to these fibres:

- at least one dye composition containing, in a medium which is suitable for dyeing, at least one oxidation base chosen from bis(phenyl)alkylenediamines, para-aminophenols, ortho-aminophenols, heterocyclic bases and para-phenylenediamines of formula (I) below, and the addition salts of these compounds with an acid:

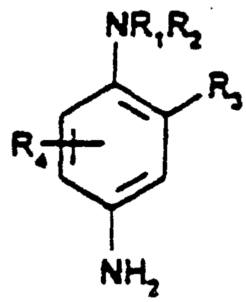

(I)

in which:

$R_1$ represents a hydrogen atom or a $C_1$-$C_4$ alkyl, $C_1$-$C_4$ monohydroxyalkyl, $C_2$-$C_4$ polyhydroxyalkyl, phenyl or 4'-aminophenyl radical,
$R_2$ represents a hydrogen atom or a $C_1$-$C_4$ alkyl, $C_1$-$C_4$ monohydroxyalkyl or $C_2$-$C_4$ polyhydroxyalkyl radical,
$R_3$ represents a hydrogen atom, a halogen atom such as a chlorine, bromine, iodine or fluorine atom, or a $C_1$-$C_4$ alkyl, $C_1$-$C_4$ monohydroxyalkyl or $C_1$-$C_4$ hydroxyalkoxy radical,
$R_4$ represents a hydrogen atom or a $C_1$-$C_4$ alkyl radical; - the colour being developed at acidic, neutral or alkaline pH using an oxidizing agent which is added, only at the time of use, to the dye composition or which is present in an oxidizing composition that is applied simultaneously or sequentially in a separate manner;

the said dye composition and/or the said oxidizing composition containing at least one ceramide-type compound.

2. Process according to Claim 1, **characterized in that** the said dye composition is mixed, at the time of use, with the said oxidizing composition containing, in a medium which is suitable for dyeing, at least one oxidizing agent present in an amount which is sufficient to develop a coloration.

3. Process according to Claim 1 or 2,
**characterized in that** the ceramide-type compound(s) is (are) chosen from natural or synthetic molecules corresponding to formula (II) below:

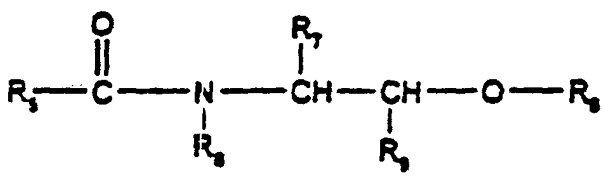

(II)

in which:

- $R_5$ denotes:
  - either a linear or branched, saturated or unsaturated, $C_5$-$C_{50}$ hydrocarbon radical, it being possible for this radical to be substituted with one or more hydroxyl groups, this or these hydroxyl group(s) optionally being esterified with an acid $R_{10}COOH$, $R_{10}$ being a saturated or unsaturated, linear or branched, optionally mono- or polyhydroxylated $C_1$-$C_{35}$ hydrocarbon radical, it being possible for the hydroxyl group(s) of the radical $R_{10}$ to be esterified with a saturated or unsaturated, linear or branched, optionally mono- or polyhydroxylated $C_1$-$C_{35}$ fatty acid,
  - or a radical R''-(NR-CO)-R', in which R denotes a hydrogen atom or a mono- or polyhydroxylated, preferably monohydroxylated $C_1$-$C_{20}$ hydrocarbon radical, R' and R'' are hydrocarbon radicals in which the sum of the carbon atoms is between 9 and 30, R' being a divalent radical,
  - or a radical $R_{11}$-O-CO-$(CH_2)_p$, in which $R_{11}$ denotes a $C_1$-$C_{20}$ hydrocarbon radical, p being an integer ranging from 1 to 12 inclusive;

- $R_6$ denotes a hydrogen atom or a $(glycosyl)_n$, $(galactosyl)_m$, sulphogalactosyl, phosphorylethylamine or phosphorylethylammonium radical, in which n is an integer ranging from 1 to 4 inclusive and m is an integer ranging from 1 to 8 inclusive;
- $R_7$ denotes a hydrogen atom or a saturated or unsaturated, hydroxylated or non-hydroxylated $C_1$-$C_{33}$ hydrocarbon radical, it being possible for the hydroxyl radical(s) to be esterified with an inorganic acid or with an acid $R_{10}COOH$, $R_{10}$ having the same meanings as those indicated above, it being possible for the hydroxyl radical(s) to be etherified with a $(glycosyl)_n$, $(galactosyl)_m$, sulphogalactosyl, phosphorylethylamine or phosphorylethylammonium radical, it also being possible for $R_7$ to be substituted with one or more $C_1$-$C_{14}$ alkyl radicals; $R_7$ preferably denotes a $C_{15}$-$C_{26}$ $\alpha$-hydroxyalkyl radical in which the hydroxyl group can optionally be esterified with a $C_{16}$-$C_{30}$ $\alpha$-hydroxy acid;
- $R_8$ denotes a hydrogen atom, a methyl or ethyl radical or a saturated or unsaturated, linear or branched, optionally hydroxylated $C_3$-$C_{50}$ hydrocarbon radical or a radical $-CH_2-CHOH-CH_2-O-R_{12}$ in which $R_{12}$ denotes a $C_{10}$-$C_{26}$ hydrocarbon radical or a radical $R_{11}-O-CO-(CH_2)_p$, $R_{11}$ denoting a $C_1$-$C_{20}$ hydrocarbon radical and p being an integer ranging from 1 to 12 inclusive;
- $R_9$ denotes a hydrogen atom or a saturated or unsaturated, linear or branched, optionally mono- or polyhydroxylated $C_1$-$C_{30}$ hydrocarbon radical, it being possible for the hydroxyl radical(s) to be etherified with a $(glycosyl)_n$, $(galactosyl)_m$, sulphogalactosyl, phosphorylethylamine or phosphorylethylammonium radical;

  with the proviso that when $R_7$ and $R_9$ denote a hydrogen atom or when $R_7$ denotes a hydrogen atom and $R_9$ denotes a methyl radical, then $R_8$ does not denote a hydrogen atom or a methyl or ethyl radical.

**4.** Process according to the preceding claim,
**characterized in that** the ceramide-type compounds are chosen from compounds of formula (II) in which $R_5$ denotes an optionally hydroxylated, saturated or unsaturated alkyl radical derived from $C_{14}$-$C_{22}$ fatty acids; $R_6$ denotes a hydrogen atom; and $R_7$ denotes an optionally hydroxylated linear, saturated $C_{11}$-$C_{17}$ radical, and preferably a $C_{13}$-$C_{15}$ radical.

**5.** Process according to the preceding claim,
**characterized in that** the ceramides are chosen from:

- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
- N-2-hydroxypalmitoyldihydrosphingosine,
- N-stearoylphytosphingosine,
- N-palmitamidohexadecanediol,

and mixtures of these compounds.

**6.** Process according to Claim 3, **characterized in that** the ceramides are chosen from compounds of formula (II) in which

- $R_5$ denotes a saturated or unsaturated alkyl radical derived from fatty acids;
- $R_6$ denotes a galactosyl or sulphogalactosyl radical;
- and $R_7$ denotes a saturated or unsaturated $C_{12}$-$C_{22}$ hydrocarbon radical and preferably a $-CH=CH-(CH_2)_{12}-CH_3$ group.

**7.** Process according to any one of the preceding claims, **characterized in that** the bis(phenyl)alkylenediamines are chosen from compounds of formula (III) below, and the addition salts thereof with an acid:

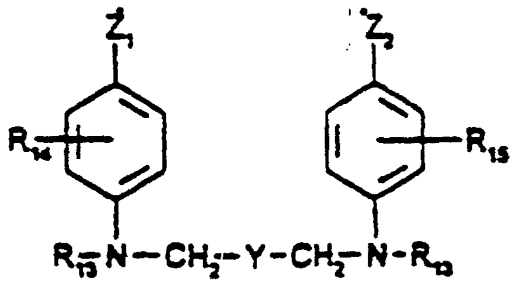

(III)

in which:

$Z_1$ and $Z_2$, which may be identical or different, represent a hydroxyl radical or a radical $NHR_{16}$ in which $R_{16}$ represents a hydrogen atom or a $C_1$-$C_4$ alkyl radical,
$R_{13}$ represents a hydrogen atom or a $C_1$-$C_4$ alkyl, $C_1$-$C_4$ monohydroxyalkyl, $C_2$-$C_4$ polyhydroxyalkyl or $C_1$-$C_4$ aminoalkyl radical in which the amino residue can be substituted,
$R_{14}$ and $R_{15}$, which may be identical or different, represent a hydrogen or halogen atom or a $C_1$-$C_4$ alkyl radical,
Y represents a radical taken from the group consisting of the following radicals:
**$-(CH_2)_n-$ : $-(CH_2)_m-O-(CH_2)_m-$ : $-(CH_2)_m-CHOH-(CH_2)_m-$** and

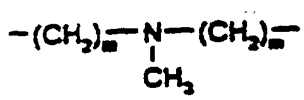 :

in which n is an integer between 0 and 8 inclusive and m is an integer between 0 and 4 inclusive.

**8.** Process according to Claim 7, **characterized in that** the bis(phenyl)alkylenediamines of formula (III) are chosen from N,N'-bis(β-hydroxyethyl)-N,N'-bis(4'-aminophenyl)-1,3-diaminopropanol, N,N'-bis(β-hydroxy-ethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylene-diamine, N,N'-bis(4-methylaminophenyl)tetramethylene-diamine, N,N'-bis(ethyl)-N,N'-bis(4'-amino-3'-methyl-phenyl)ethylenediamine, and the addition salts thereof with an acid.

**9.** Process according to any one of the preceding claims, **characterized in that** the para-aminophenols are chosen from compounds corresponding to formula (IV) below, and the addition salts thereof with an acid:

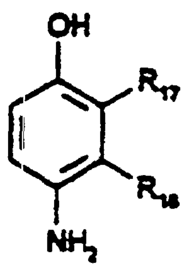

(IV)

in which:

$R_{17}$ represents a hydrogen atom or a $C_1$-$C_4$ alkyl, $C_1$-$C_4$ monohydroxyalkyl, ($C_1$-$C_4$) alkoxy ($C_1$-$C_4$) alkyl, $C_1$-$C_4$ aminoalkyl or hydroxy ($C_1$-$C_4$) alkylamino ($C_1$-$C_4$) alkyl radical,
$R_{18}$ represents a hydrogen or fluorine atom or a $C_1$-$C_4$ alkyl, $C_1$-$C_4$ monohydroxyalkyl, $C_2$-$C_4$ polyhydroxyalkyl, $C_1$-$C_4$ aminoalkyl, cyano ($C_1$-$C_4$)alkyl or ($C_1$-$C_4$)alkoxy-($C_1$-$C_4$) alkyl radical,

it being understood that at least one of the radicals $R_{17}$ and $R_{18}$ represents a hydrogen atom.

**10.** Process according to Claim 9, **characterized in that** the para-aminophenols of formula (IV) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol and 4-amino-2-(β-hydroxyethylaminomethyl)phenol, and the addition salts thereof with an acid.

**11.** Process according to any one of the preceding claims, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

**12.** Process according to any one of the preceding claims, **characterized in that** the heterocyclic bases are chosen from pyridine derivatives, pyrimidine derivatives and pyrazole derivatives, and the addition salts thereof with an acid.

**13.** Process according to Claim 12, **characterized in that** the heterocyclic oxidation bases are chosen from 2,4,5,6-tetraaminopyrimidine, 2,5-diaminopyridine, 4,5-diamino-1-methylpyrazole, 3,4-diaminopyrazole, 4,5-diamino-1-(4'-chlorobenzyl)pyrazole and 4-hydroxy-2,5,6-triaminopyrimidine, and the addition salts thereof with an acid.

**14.** Process according to any one of the preceding claims, **characterized in that** the para-phenylenediamines of formula (I) are chosen from paraphenylenediamine, para-toluylenediamine, 2-chloro-paraphenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis (β-hydroxyethyl) -para-phenylenediamine, 4-amino-N,N-bis(β-hydroxyethyl)-3-methylaniline, 4-amino-3-chloro-N,N-bis(β-hydroxyethyl)aniline, 2-β-hydroxyethyl-para-phenylenediamine, 2-fluoro-para-phenylene-diamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl, β-hydroxyethyl)-para-phenylenediamine, N-(β,γ-dihydroxypropyl)-para-phenylenediamine, N-(4'-aminophenyl)-para-phenylene-diamine, N-phenyl-para-phenylenediamine and 2-β-hydroxyethyloxy-para-phenylenediamine, and the addition salts thereof with an acid.

**15.** Process according to Claim 14, **characterized in that** the para-phenylenediamines of formula (I) are chosen from para-phenylenediamine, para-toluylene-diamine, 2-isopropyl-para-phenylenediamine, 2-β-hydroxyethyl-para-phenylenediamine, 2-β-hydroxyethyl-oxy-para-phenylenediamine, 2,6-dimethyl-para-phenylenediamine, 2,6-diethyl-para-phenylenediamine, 2,3-dimethyl-para-phenylenediamine, N,N-bis (β-hydroxyethyl)-para-phenylenediamine and 2-chloro-para-phenylenediamine, and the addition salts thereof with an acid.

**16.** Process according to any one of the preceding claims, **characterized in that** the ceramide-type compound(s) represent(s) from 0.0001% to 10% by weight relative to the total weight of the dye composition or relative to the total weight of the oxidizing composition.

**17.** Process according to Claim 16, **characterized in that** the ceramide-type compound(s) represent(s) from 0.001% to 5% by weight relative to the total weight of the dye composition or relative to the total weight of the oxidizing composition.

**18.** Process according to any one of the preceding claims, **characterized in that** the oxidation base(s) represent(s) from 0.0001 to 20% by weight relative to the total weight of the dye composition.

**19.** Process according to Claim 17, **characterized in that** the oxidation base(s) represent(s) from 0.005 to 10% by weight relative to the total weight of the dye composition.

**20.** Process according to any one of the preceding claims, **characterized in that** the dye composition also contains one or more couplers chosen from metadiphenols, meta-aminophenols, meta-phenylenediamines, mono- or polyhydroxylated naphthalene derivatives, sesamol and its derivatives, heterocyclic compounds such as, for example, pyridine couplers and indole couplers, and the addition salts thereof with an acid.

**21.** Process according to Claim 20, **characterized in that** the coupler(s) represent(s) from 0.0005% to 20% by weight relative to the total weight of the dye composition.

**22.** Process according to Claim 21, **characterized in that** the coupler(s) represent(s) from 0.01% to 10% by weight

relative to the total weight of the dye composition.

**23.** Process according to any one of the preceding claims, **characterized in that** the addition salts with an acid are chosen from the hydrochlorides, hydrobromides, sulphates and tartrates.

**24.** Process according to any one of the preceding claims, **characterized in that** the medium which is suitable for dyeing (or the support) consists of water or a mixture of water and at least one organic solvent chosen from $C_1$-$C_4$ lower alkanols, glycerol, glycols and glycol ethers, aromatic alcohols, similar products and mixtures thereof.

**25.** Process according to any one of the preceding claims, **characterized in that** the dye composition has a pH of between 3 and 11.5.

**26.** Process according to any one of the preceding claims, **characterized in that** the oxidizing agent is chosen from hydrogen peroxide, urea peroxide, alkali metal bromates and persalts such as perborates and persulphates.

**27.** Composition for the oxidation dyeing of keratin fibres, and in particular human keratin fibres such as the hair, **characterized in that** it comprises, in a medium which is suitable for dyeing:

- at least one oxidation base chosen from bis(phenyl)alkylenediamines, para-aminophenols, ortho-aminophenols, heterocyclic bases and para-phenylenediamines of formula (I) below, and the addition salts of these compounds with an acid:

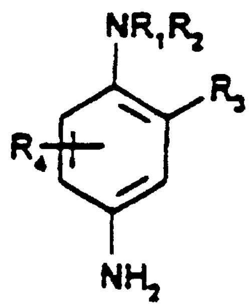

(I)

in which:

$R_1$ represents a hydrogen atom or a $C_1$-$C_4$ alkyl, $C_1$-$C_4$ monohydroxyalkyl, $C_2$-$C_4$ polyhydroxyalkyl, phenyl or 4'-aminophenyl radical,
$R_2$ represents a hydrogen atom or a $C_1$-$C_4$ alkyl, $C_1$-$C_4$ monohydroxyalkyl or $C_2$-$C_4$ polyhydroxyalkyl radical,
$R_3$ represents a hydrogen atom, a halogen atom such as a chlorine, bromine, iodine or fluorine atom, or a $C_1$-$C_4$ alkyl, $C_1$-$C_4$ monohydroxyalkyl or $C_1$-$C_4$ hydroxyalkoxy radical,
$R_4$ represents a hydrogen atom or a $C_1$-$C_4$ alkyl radical,

- and at least one ceramide-type compound.

**28.** Multi-compartment device or multi-compartment dyeing "kit", a first compartment of which contains a dye composition containing, in a medium which is suitable for dyeing, at least one oxidation base chosen from bis(phenyl) alkylenediamines, para-aminophenols, ortho-aminophenols, heterocyclic bases and para-phenylenediamines of formula (I) below, and the addition salts of these compounds with an acid:

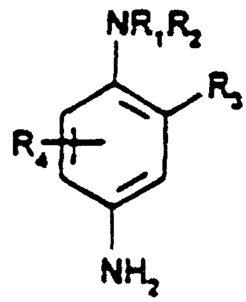

(I)

in which:

$R_1$ represents a hydrogen atom or a $C_1$-$C_4$ alkyl, $C_1$-$C_4$ monohydroxyalkyl, $C_2$-$C_4$ polyhydroxyalkyl, phenyl or 4'-aminophenyl radical,
$R_2$ represents a hydrogen atom or a $C_1$-$C_4$ alkyl, $C_1$-$C_4$ monohydroxyalkyl or $C_2$-$C_4$ polyhydroxyalkyl radical,
$R_3$ represents a hydrogen atom, a halogen atom such as a chlorine, bromine, iodine or fluorine atom, or a $C_1$-$C_4$ alkyl, $C_1$-$C_4$ monohydroxyalkyl or $C_1$-$C_4$ hydroxyalkoxy radical,
$R_4$ represents a hydrogen atom or a $C_1$-$C_4$ alkyl radical, and a second compartment of which contains an oxidizing composition;

the said dye composition and/or the said oxidizing composition containing at least one ceramide-type compound.